# EUROPEAN PATENT SPECIFICATION

(11) **EP 1 944 000 B1**
(45) Date of publication and mention of the grant of the patent: **11.08.2010**
(21) Application number: 08000408.8
(22) Date of filing: 10.01.2008
(51) Int. Cl.: A61H 23/02

(54) **Skin care device**
Hautpflegevorrichtung
Dispositif de soins cutanés

(30) Priority: 15.01.2007 JP 2007006243
(43) Date of publication of application: 16.07.2008
(73) Proprietor: Panasonic Electric Works Co., Ltd., Kadoma-shi Osaka (JP)
(72) Inventor: Matsusaka, Takeshi, Kadoma-shi Osaka (JP); Fujiwara, Mitsuru, Kadoma-shi Osaka (JP)
(74) Representative: Samson & Partner

(56) References cited:
- WO-A-02/076552
- JP-A- 2006 034 651
- US-A- 5 086 788

## Description

The present invention relates to a skin care device according to the preamble of claim 1. Such a skin care device is known from JP 2006 204767A. Further, document WO 02/076552 A discloses an osteoporosis treatment apparatus comprising a head block having an ultrasonic vibrator generating ultrasonic vibration, a grip block for gripping a rail and supporting the head block, the head block being pressed against a skin surface thereby transmitting the ultrasonic vibration to the skin surface, and an elastic member serving as a support mechanism for supporting the head block on the grip block, wherein the head block is supported on the grip block through the elastic member in such a way that the head block is allowed to be three-dimensionally rotated in all directions with respect to the grip block and also to be moved toward and away from the grip block.

Conventionally, there is known a skin care device that can attain a desired aesthetic effect by pressing a head block against a skin surface and then transmitting the ultrasonic vibration of an ultrasonic vibrator provided in the head block to the skin surface. The head block of the skin care device is supported on one longitudinal end portion of a grip block that has a control circuit and an operation switch. The skin care device is of a structure in which the ultrasonic vibrator in the head block converts electric vibration supplied from the control circuit in the grip block to mechanical vibration, thereby generating ultrasonic vibration.

In the skin care device referred to above, it is preferred that, if there exist irregularities on the skin surface against which the head block is pressed, the head block vibrates while conforming to the irregularities. Therefore, Japanese Patent Laid-open Application No. 2006-204767 (JP2006-204767A) discloses a skin care device in which a head block is rotatably supported on a grip block so that the head block can rotate about an intersection point of two axes lying at a right angle. In this skin care device, the head block is three-dimensionally rotatable about the point at which the two axes intersect.

The head block of the skin care device is designed to merely rotate about one point with respect to the grip block. For this reason, in case the head block is pressed against, e.g., a skin surface having large irregularities to be slid therealong, it is difficult for the head block to smoothly conform to the irregularities during its movement.

Also described in JP2006-204767A is a technique for making the head block floatingly movable. The floating movement of the head block is realized by basically using a mechanism for supporting the head block in such a way that it can rotate about one point and additionally employing a mechanism for allowing the head block to be slid in a specified direction. In this case, since the rotatable movement of the head block is made about one point and the direction of sliding movement is confined to a specified direction, it is hard to say that the head block can smoothly conform to the irregularities of the skin surface. Moreover, the mechanisms are complex and expensive, which makes it difficult to provide a skin care device in a cost-effective manner.

In view of the problems mentioned above, the present invention provides a skin care device capable of allowing a head block to smoothly conform to the irregularities of a skin surface and also capable of being manufactured in a cost-effective manner.

In accordance with the present invention, this problem will be solved by a skin care device including the features of claim 1.

By employing a structure in which the head block is supported on the grip block through the elastic member, the head block is three-dimensionally rotatable in all directions with respect to the grip block and also movable toward and away from the grip block as a result of the crushing and bending deformation of the elastic member. Unlike the prior art configuration, the head block does not pivotably move about a fixed point but makes pivotable movement about an arbitrary point suitably decided depending on the shape of the skin surface against which the head block is pressed. Therefore, even when the skin surface has large irregularities, the head block can quickly move along the skin surface in a variety of postures and can perform skin care while smoothly conforming to the skin surface. In addition, this configuration is simple and can be provided in a cost-effective manner.

In the inventive skin care device configured according to claim 1 the elastic member normally imparts a biasing force on the head block in a direction that the head block is moved away from the grip block. The stopper of the head block is pressed against the inner surface of the housing by the biasing force, thereby maintaining the head block in a specified standard posture. Owing to the fact that the elastic member imparts a biasing force equal to or grater than a prescribed level on the head block from the initial pressing time, it is possible to ensure that the head block closely conforms to and contacts with the skin surface.

In the skin care device provided with the stopper, it is preferred that a water-proof cover is interposed between the housing of the grip block and the stopper. By doing so, the liquid such as gel or the like used as an ultrasonic wave transmitting medium during skin care is prevented from infiltrating into the grip block, thereby avoiding circuit breakage or other troubles in a reliable manner.

It is also preferred that the skin care device configured as above further includes a water-proof cover interposed between the housing of the grip block and the stopper. Accordingly, the liquid such as gel or the like used as an ultrasonic wave transmitting medium for skin care is prevented from infiltrating into the grip block, thereby avoiding circuit breakage or other troubles in a reliable manner.

In the skin care device configured as above, it is preferred that the head block has a bottom surface formed of a spherical convex surface and the grip block has a top surface formed of a spherical concave surface facing the convex surface of the head block and wherein a gap for permitting movement of the head block is provided between the convex surface of the head block and the concave surface of the grip block. This ensures that, if the head block is pushed inwardly by an extent corresponding to the gap, the convex surface forming the bottom surface of the head block contacts with the concave surface forming the top surface of the grip block, at which time the sinking movement of the head block is restricted. In this state, the convex surface of the head block is slidably fitted to the concave surface, whereby the head block can make three-dimensional rotatable movement in all directions under the guidance of the concave surface. In case an ultrasonic wave transmitting medium such as gel or the like adheres to the bottom surface of the head block and remains behind, the residual medium can be easily dislodged by inserting a cotton stick or the like into the gap.

In accordance with the present invention, a skin care device capable of allowing a head block to smoothly conform to the irregularities of a skin surface can be provided in a cost-effective manner.

The objects and features of the present invention will become apparent from the following description of preferred embodiments given in conjunction with the accompanying drawings, in which:
Fig. 1 is a side cross sectional view showing a skin care device in accordance with an embodiment of the present invention.
Figs. 2A and 2B are front and rear views illustrating the skin care device.
Fig. 3 is an exploded perspective view showing a grip block of the skin care device.

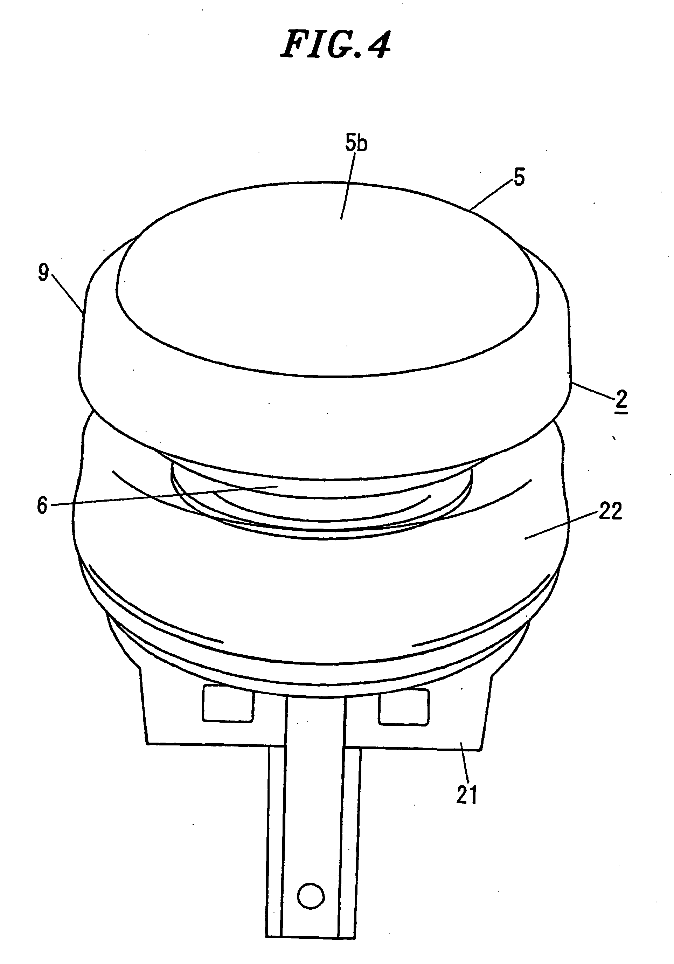

Fig. 4 is a perspective view showing characterized parts of the skin care device.
Fig. 5 is an exploded perspective view showing the characterized parts of the skin care device.
Fig. 6 is a side cross sectional view showing the characterized parts of the skin care device.
Fig. 7 is a side cross sectional view showing the characterized parts of the skin care device, with a head block being in a pushed-in state.
Fig. 8 is a side cross sectional view showing the characterized parts of a skin care device in accordance with a modified example of the embodiment of the present invention.

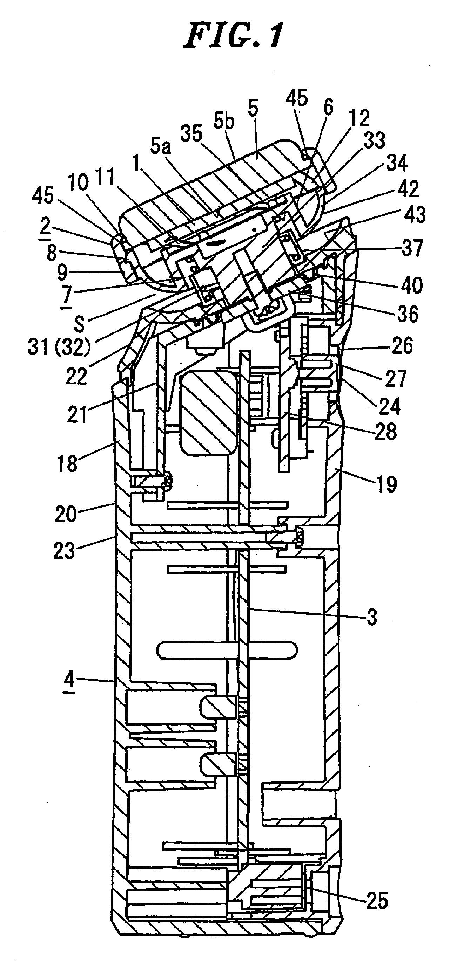

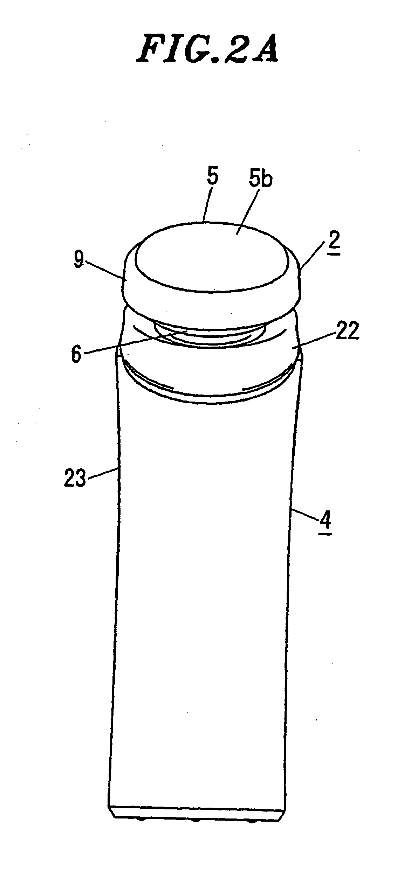

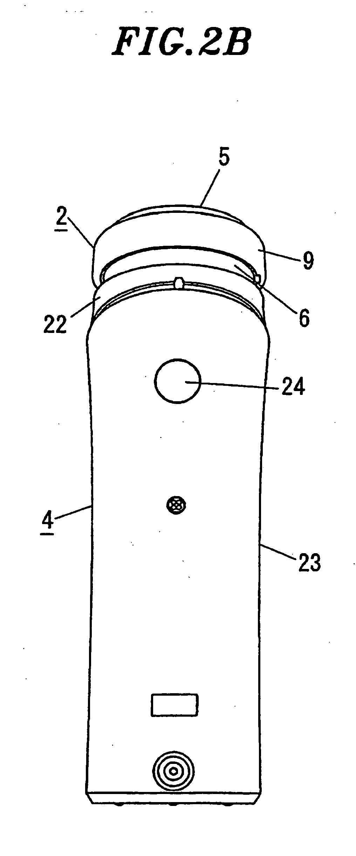

Hereinafter, the present invention will be described with reference to the embodiment shown in the accompanying drawings. Figs. 1, 2A and 2B show the whole view of a skin care device in accordance with an embodiment of the present invention. The skin care device in accordance with the embodiment of the present invention includes, as its major components, a head block 2 with a built-in ultrasonic vibrator 1 and a grip block 4 having a built-in control circuit 3 that supplies electric vibration to the ultrasonic vibrator 1 to generate ultrasonic vibration.

First, description will be made on the basic configuration of the head block 2. The head block 2 includes a horn 5 of disc shape made of metal such as aluminum or the like, an ultrasonic vibrator 1 adhesively fixed to one flat surface of the horn 5, a head base 6 forming the bottom portion of the head block 2, and a ring-shaped water-proof cover 9 that engages with a flange-like support 45 projecting from the peripheral surface of the horn 5 and, at the same time, engages with a hook-like engagement part 8 protruding from the outer surface of the head base 6. By attaching and fixing the horn 5 to the head base 6 through the water-proof cover 9, the flat surface (hereinafter called an "ultrasonic wave emitting surface 5b") of the horn 5 opposite to the flat surface (hereinafter called a "connection surface 5a") to which the ultrasonic vibrator 1 is fixed can be exposed to the outside in a state that it protrudes obliquely and upwardly as viewed in the figure. An O-ring 10 is interposed between the support 45 of the horn 5 and the water-proof cover 9.

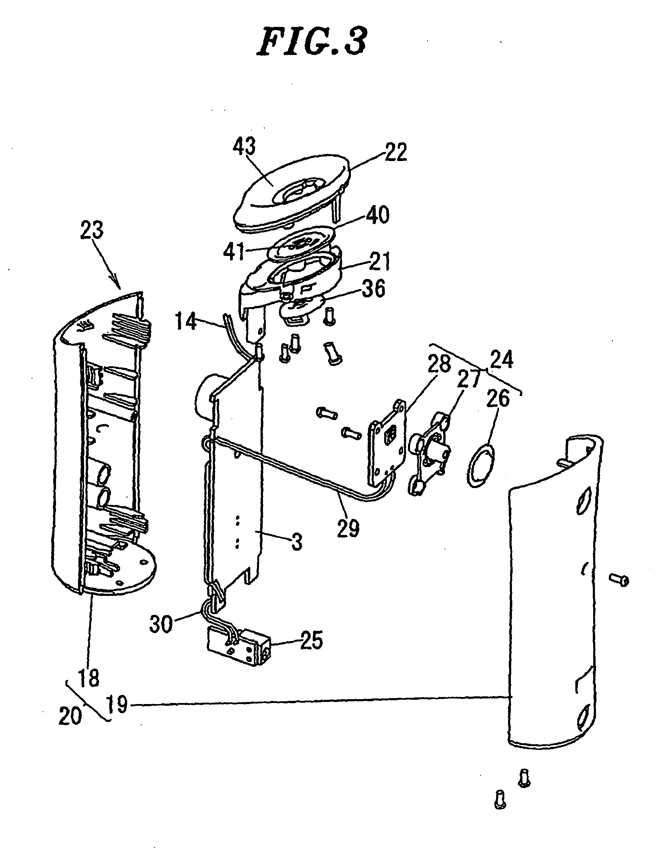

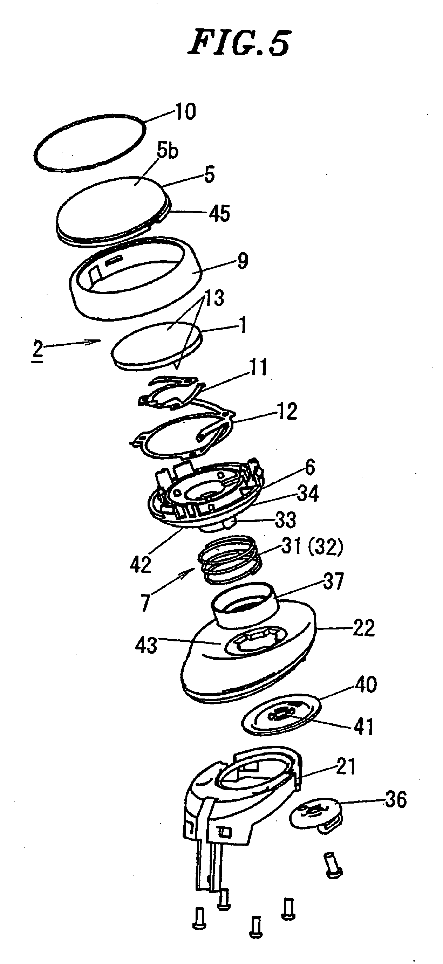

Further, provided in the head block 2 are a vibrator-side connection terminal 11 and a horn-side connection terminal 12 for supplying electricity to the ultrasonic vibrator 1. The vibrator-side connection terminal 11 makes an elastic contact with one of a pair of electrodes 13 (see Fig. 5) provided in the ultrasonic vibrator 1, whereby the vibrator-side connection terminal 11 is directly and electrically connected to the corresponding electrode 13. Furthermore, the horn-side connection terminal 12 makes an elastic contact with the connection surface 5a of the horn 5, whereby the horn-side connection terminal 12 is electrically connected to the other electrode 13 of the ultrasonic vibrator 1. The connection terminals 11 and 12 are both fixed to the head base 6 and electrically connected to the control circuit 3 in the grip block 4 via a lead line 14 (see Fig. 3).

Next, description will be made on the basic configuration of the grip block 4. As shown in Fig. 3, the grip block 4 includes a bottom-closed tubular main housing 20 having a front housing 18 and a rear housing 19 screw-fixed to the front housing 18, a generally ring-shaped support base 21 screw-fixed to the opening of the main housing 20 and a top cover 22. The main housing 20, the support base 21 and the top cover 22 form an overall housing 23 of the grip block 4.

Installed in the housing 23 are the control circuit 3, an operation switch 24 and a power source jack 25. The operation switch 24 includes a switch cover 26 attached to the rear housing 19, a switch boss 27 and a switch substrate 28. The switch boss 27 and the switch substrate 28 are screw-fixed to the rear housing 19. The switch substrate 28 and the control circuit 3 are electrically connected to each other via lead lines 29. The power source jack 25 and the control circuit 3 are electrically connected to each other via lead lines 30.

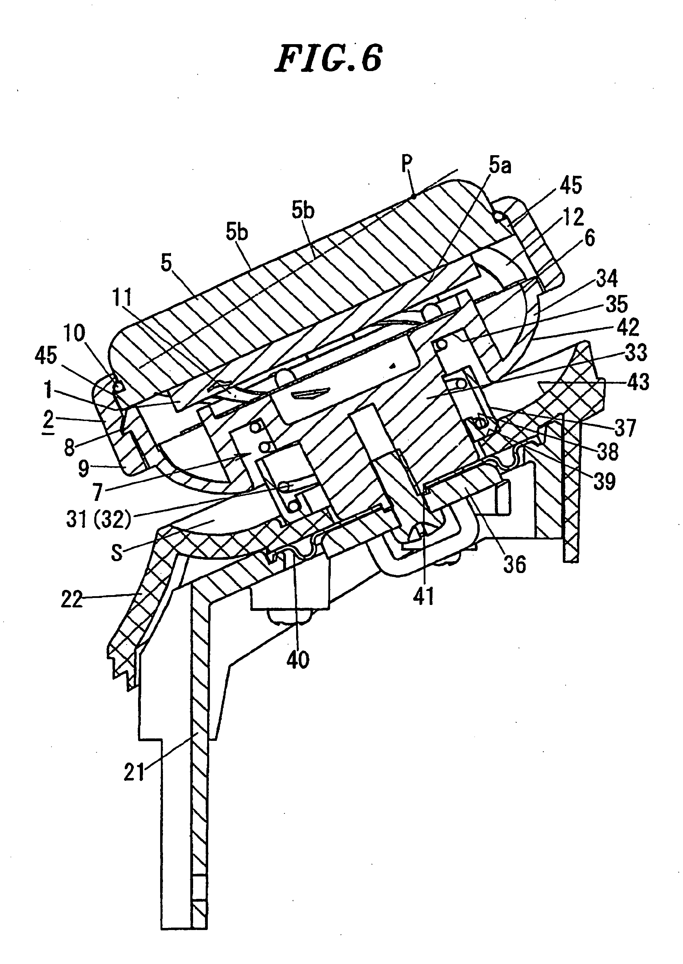

Next, description will be made on a support mechanism 7 for movably supporting the head block 2 on the grip block 4. As illustrated in Figs. 5 and 6, the support mechanism 7 employed in the embodiment of the present invention includes a coil spring 32 as an elastic member 31 interposed between the head base 6 forming the bottom portion of the head block 2 and the top cover 22 forming the top portion of the grip block 4. The bottom of the head base 6 includes a central columnar penetration portion 33, a peripheral portion 34 surrounding the penetration portion 33, and an annular groove portion 35 formed between the penetration portion 33 and the peripheral portion 34. The penetration portion 33 is inserted into the central aperture of the top cover 22 while a disc-like stopper 36 is screw-fixed to the tip end of the penetration portion 33 within the top cover 22 (i.e., within the housing 23). The stopper 36 is a member that prevents the head block 2 from slipping through by contacting with the inner surface of the top cover 22 through a water-proof cover 40 which will be described below.

The coil spring 32 forming the support mechanism 7 is inserted into the groove portion 35 of the head base 6 at one end thereof and is contacted with the top cover 22 at the other end thereof. The coil spring 32 is kept compressed at all times by means of the stopper 36 that prevents the head block 2 from slipping through. A cylindrical water-proof member 37 that surrounds the outer circumference of the coil spring 32 is further provided in the embodiment of the present invention. The above-described other end of the coil spring 32 is retained within an engagement groove 38 formed in the end portion of the water-proof member 37 and, the coil spring 32 is pressed against the top cover 22 through the water-proof member 37. In the portion of the top cover 22 surrounding the central opening, there is formed a step portion 39 to which the end portion of the water-proof member 37 is fitted. Therefore, the water-proof member 37 is pressed against and fitted to the step portion 39 by the biasing force of the compressed coil spring 32, whereby the water-proof member 37 can be securely kept in a specified position surrounding the coil spring 32.

The water-proof member 37 is made of a material such as EPDM (Ethylene propylene diene monomer), NBR (Nitile Butadien Rubber), silicon rubber or the like. The coil spring 32 is made of a material of a cold coiling spring such as SWPA, SWPB, SUS304, SUS304WPB or the like and is formed to have an inner diameter of 16.0 mm.

A sheet-shaped water-proof cover 40 made of a material such as EPDM, NBR, silicon rubber or the like is arranged inside the top cover 22 of the grip block 4. The outer peripheral part of the water-proof cover 40 is embedded and fixed between the top cover 22 and the support base 21. The water-proof cover 40 is provided with a through hole 41 at the center thereof. At the peripheral portion of the through hole 41, the center part of the water proof cover 40 is embedded and fixed between the penetration portion 33 of the head base 6 and the stopper 36. Thus, the water-proof cover 40 is adapted to be interposed between the housing 23 of the grip block 4 and the stopper 36.

In the embodiment of the present embodiment, the tubular water-proof member 37 attached to the coil spring 32 and the sheet-shaped water-proof cover 40 attached to the inner surface of the top cover 22 make sure that the liquid such as gel or the like used as an ultrasonic wave transmitting medium during skin care is prevented from infiltrating into the grip block 4, thereby avoiding circuit breakage or other troubles in a reliable manner. Further, the O-ring 10 arranged between the horn 5 and the water-proof cover 9 prevents the liquid such as gel or the like from infiltrating into the head block 2.

In the head base 6 forming the bottom portion of the head block 2, the outer surface of the peripheral portion 34 surrounding the groove portion 35 on which the coil spring 32 rests is formed of a spherically-curved convex surface 42. The outer surface of the top cover 22 forming the top portion of the grip block 4 is formed of a spherically-curved concave surface 43 that is likely to be fitted to the convex surface 42. In other words, the head block 2 can make rotatable movement about any point, or at least each of multiple points, on the horn 5 of the head block 2, at which point the head block 2 make contact with the skin surface. For example, the convex surface 42 forms the bottom surface of the head block 2, while the concave surface 43 forms the top surface of the grip block 4 facing the convex surface 42. Due to the presence of the coil spring 32, a specified gap S is left between the convex surface 42 and the concave surface 43.

With the skin care device in accordance with the embodiment of the present invention configured as above, if the operation switch 24 is operated to turn on an electric power source, electric vibration is supplied from the control circuit 3 to the ultrasonic vibrator 1 via the lead lines 14 and the connection terminals 11 and 12. The ultrasonic vibrator 1 converts the electric vibration to mechanical vibration to thereby generate ultrasonic vibration. Then, the ultrasonic wave emitting surface 5b of the horn 5 is pressed against a skin surface while gripping the grip block 4 with one hand, so that the ultrasonic vibration generated in the ultrasonic vibrator 1 is transmitted to the skin surface or other destinations of a living body, thus attaining a desired aesthetic effect. It is preferred that an ultrasonic wave transmitting medium such as gel or the like is placed between the ultrasonic wave emitting surface 5b of the horn 5 and the skin surface.

As described above, the skin care device of the present invention is of a structure wherein the coil spring 32 is used as the support mechanism 7 for movably supporting the head block 2 on the grip block 4 and the coil spring 32 is kept in a compressed state by allowing the stopper 36 fixed to the bottom portion of the head block 2 to engage with the inner surface of the housing 23 of the grip block 4. Consequently, the coil spring 32 normally imparts a biasing force on the head block 2 in a direction that the head block 2 is moved away from the grip block 4. The stopper 36 of the head block 2 is pressed against the inner surface of the housing 23 by the biasing force, thereby maintaining the head block 2 in a specified standard posture as illustrated in Fig. 6. In the standard posture, the inclined angle that the ultrasonic wave emitting surface 5b makes with respect to the surface orthogonal to the axial direction of the grip block6 may be set in a range of 5 to 40 degrees, preferably 9 to 35 degrees and more preferably 25 to 35 degrees.

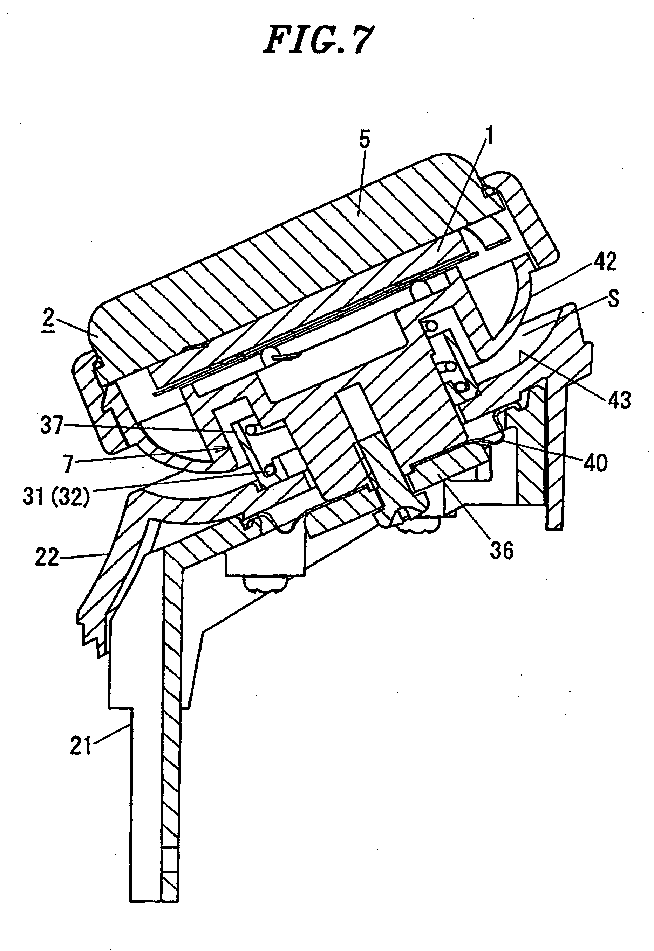

For example, when the ultrasonic wave emitting surface 5b of the horn 5 is pressed in the direction perpendicular to the skin surface while being moved along the skin surface, the head block 2 gets closer to the grip block 4 while crushing the coil spring 32 as the support mechanism 7 in its axial direction. Owing to the fact that the coil spring 32 is initially in a compressed state, a biasing force equal to or grater than a prescribed level is applied to the horn 5 from the initial pressing time. The application of the biasing force makes it possible to bring the ultrasonic wave emitting surface 5b of the horn 5 into close contact with the skin surface. Fig. 7 illustrates a case in which the head block 2 is sunk toward the grip block 4 by 1.5 mm.

The mounting load of the coil spring 32 is preferably in a range of 0.5 to 2.0 N and more preferably in a range of 1.0 to 1.5 N. When the horn 5 is pressed against the skin surface, the biasing force of the coil spring 32 imparted on the horn 5 is preferably in a range of 2.0 to 4.0 N and more preferably in a range of 2.5 to 3.0 N.

If the ultrasonic wave emitting surface 5b of the horn 5 is pressed to the skin surface in a direction slanted from the direction orthogonal to the skin surface while being moved along the skin surface, the head block 2 is three-dimensionally rotated along the skin surface while bending the coil spring 32 as the support mechanism 7. Since the coil spring 32 is in a compressed state at the outset, a biasing force equal to or grater than a prescribed level is applied to the horn 5 from the initial pressing time. Therefore, the application of the biasing force makes it possible to bring the ultrasonic wave emitting surface 5b of the horn 5 into close contact with the skin surface.

Since the crushing and bending deformation of the coil spring 32 occurs in combination as set forth above, the head block 2 is free to move toward and away from the grip block 4 and also free to three-dimensionally rotate in all directions with respect to the grip block 4. Therefore, even when the skin surface has large irregularities, the head block 2 can quickly move along the skin surface in a variety of postures and can perform skin care while smoothly conforming to the skin surface.

Unlike the prior art, the head block 2 in accordance with the embodiment of the present invention does not rotate about a fixed point but makes rotatable movement about an arbitrary point suitably decided depending on the shape of the skin surface against which the head block 2 is pressed. In other words, the head block 2 can make roratable movement about any one of multiple points on the horn 5 of the head block 2, at which point the head block 2 make contact with the skin surface. For example, when the point P becomes a center of the pivotal movement as illustrated in Fig. 6, the horn 5 is rotated in such a manner that the ultrasonic wave emitting surface 5b can be tilted as indicated by a dotted line. The extent over which the head block 2 can be sunk is preferably set to be in a range of 2.0 to 6.0 mm and more preferably in a range of 3.0 to 5.0 mm. Further, the angle at which the head block 2 can be rotated is preferably set to be in a range of 5 to 15 degrees and more preferably in a range of 8 to 12 degrees.

Although not specifically illustrated, if the head block 2 is pushed inwardly by an extent corresponding to the gap S, the convex surface 42 forming the bottom surface of the head block 2 contacts with the concave surface 43 forming the top surface of the grip block 4, at which time the sinking movement of the head block 2 is restricted. In this state, the convex surface 42 of the head block 2 is slidably fitted to the concave surface 43, whereby the head block 2 can make three-dimensional rotatable movement in all directions under the guidance of the concave surface 43.

Further, in the event that an ultrasonic wave transmitting medium such as gel or the like is used for the skin care process, it is sometimes the case that the medium adheres to the bottom surface of the head block 2 and remains behind. Even in the case, the gap S left between the head block 2 and the grip block 4 provides an advantage in that the residual medium can be easily dislodged by inserting a cotton stick or the like into the gap S.

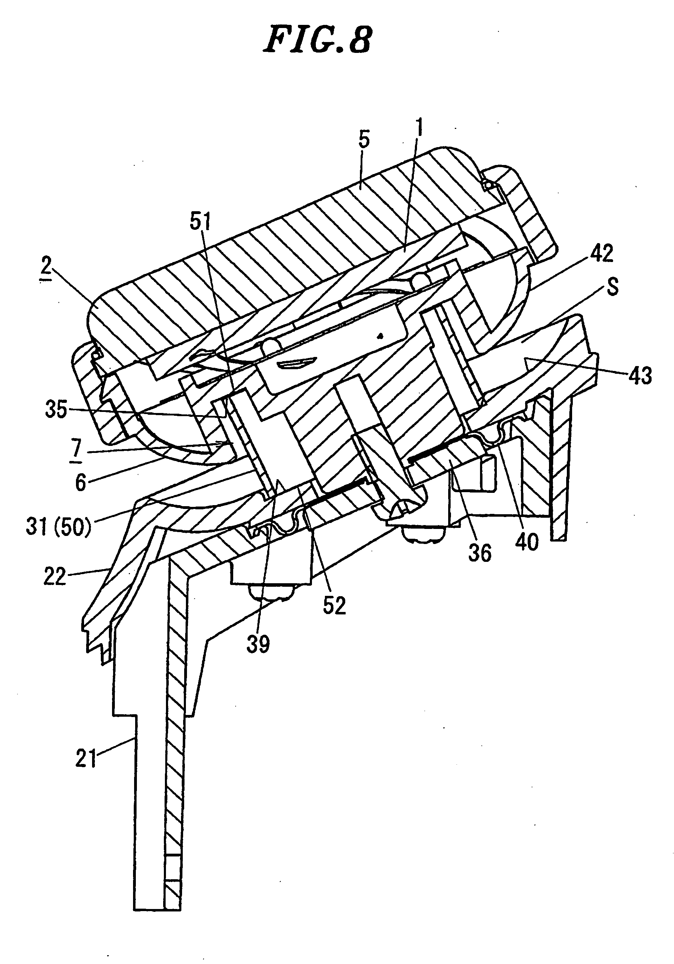

Shown in Fig. 8 is a skin care device in accordance with a modified example of the present invention. The basic configuration employed in this example is the same as that of the preceding example. Therefore, only the characterized configuration will be described below, with no description given to the same configuration.

In this example, a tubular member 50 of which opposite ends are opened is used as an elastic member 31 forming the support mechanism 7. The tubular member 50 is made of a material such as EPDM, NBR, silicon rubber or the like. The circumferential portion 51 around one end opening of the tubular member 50 contacts with the bottom surface of the groove portion 35 of the head base 6, whereas the circumferential portion 52 around the other end opening of the tubular member 50 contacts with the step portion 39 of the top cover 22. This provides a structure in which the head block 2 is supported on the grip block 4 through the tubular member 50.

As the preceding example, use of the tubular member 50 allows the head block 2 to be supported on the grip block 4 in such a way that the head block 2 can be three-dimensionally rotated in all directions with respect to the grip block 4 and also can be moved toward and away from the grip block 4. As compared to the preceding example, use of the tubular member 50 makes it possible to further reduce the number of components and to provide a skin care device in a cost-effective manner.

While the invention has been shown and described with respect to the preferred embodiments, it will be understood by those skilled in the art that various changes and modifications may be made without departing from the scope of the invention as defined in the following claims.

## Claims

1. A skin care device comprising:
a head block (2) having an ultrasonic vibrator (1) generating ultrasonic vibration;
a grip block (4) supporting the head block (2), the head block (2) being pressed against a skin surface, thereby transmitting the ultrasonic vibration to the skin surface; and
a joint member (31) serving as a support mechanism (7) for supporting the head block (2) on the grip block (4),
wherein the head block (2) is supported on the grip block (4) through the joint member (31) in such a way that the head block (2) is allowed to be moved toward and away from the grip block (4),
**characterized in that**
- the joint member (31) serving to further allow the head block (2) to be three-dimensionally rotated in all directions with respect to the grip block (4), and
- a slipping through-proof stopper (36) is coupled to a bottom portion of the head block (2), the slipping through-proof stopper (36) being inserted into a housing (20) of the grip block (4) and caught by the inner surface of the housing (20), whereby the joint member is an elastic member (31) which is kept in a compressed state to allow the elastic member (31) to exert a biasing force in such a direction as to move the head block (2) away from the grip block (4).

2. The skin care device of claim 1, wherein a water-proof cover (40) is interposed between the housing (20) of the grip block (4) and the stopper (36).

3. The skin care device of claim 1 or 2, further comprising a water-proof member (37) for covering an outer circumference of the elastic member (31) serving as the support mechanism (7).

4. The skin care device of any one of claims 1-3, wherein the head block_(2) has a bottom surface formed of a spherical convex surface (42) and the grip block (4) has a top surface formed of a spherical concave surface (43) facing the convex surface (42) of the head block (2) and wherein a gap (S) permitting movement of the head block (2) is provided between the convex surface (42) of the head block (2) and the concave surface (43) of the grip block (4).

## Patentansprüche

1. Hautpflegevorrichtung, umfassend:
einen Kopfblock (2), der einen Ultraschallvibrator (1) aufweist, der Ultraschallvibration erzeugt;
einen Griffblock (4), der den Kopfblock (2) trägt, wobei der Kopfblock (2) gegen eine Hautoberfläche gedrückt wird, wodurch die Ultraschallvibration auf die Hautoberfläche übertragen wird; und
ein Gelenkelement (31), das als ein Trageelement (7) zum Tragen des Kopfblocks (2) auf dem Griffblock (4) dient,
wobei der Kopfblock (2) auf dem Griffblock (4) durch das Gelenkelement (31) derart getragen wird, dass dem Kopfblock (2) erlaubt wird, in Richtung und weg vom Griffblock (4) bewegt zu werden,
**dadurch gekennzeichnet, dass**
- das Gelenkelement (31) dazu dient, ferner dem Kopfblock (2) zu erlauben, dreidimensional in alle Richtungen bezüglich des Griffblocks (4) zu rotieren, und
- ein durchrutschfester Stopper (36) an einen Bodenabschnitt des Kopfblocks (2) gekoppelt ist, wobei der durchrutschfeste Stopper (36) in ein Gehäuse (20) des Griffblocks (4) eingeführt und von der Innenfläche des Gehäuses (20) erfasst wird, wobei das Gelenkelement ein elastisches Element (31) ist, das in einem komprimierten Zustand gehalten wird, um dem elastischen Element (31) zu erlauben, eine Vorspannkraft in einer derartigen Richtung auszuüben, um den Kopfblock (2) von dem Griffblock (4) wegzubewegen.

2. Hautpflegevorrichtung gemäß Anspruch 1, wobei ein wasserfestes Gehäuse (40) zwischen das Gehäuse (20) des Griffblocks (4) und den Stopper (36) gestellt ist.

3. Hautpflegevorrichtung gemäß Anspruch 1 oder 2, welche ferner ein wasserfestes Element (37) umfasst zum Bedecken eines Außenumfangs des elastischen Elements (31), das als der Tragemechanismus (7) dient.

4. Hautpflegevorrichtung gemäß einem der Ansprüche 1-3, wobei der Kopfblock (2) eine Bodenoberfläche aufweist, die aus einer kugelförmigen konvexen Oberfläche (42) gebildet ist, und der Griffblock (4) eine obere Oberfläche aufweist, die aus einer kugelförmigen konkaven Oberfläche (43) gebildet ist, die der konvexen Oberfläche (42) des Kopfblocks (2) gegenüberliegt, und wobei ein Spalt (S), der eine Bewegung des Kopfblocks (2) erlaubt, zwischen der konvexen Oberfläche (42) des Kopfblocks (2) und der konkaven Oberfläche (43) des Griffblocks (4) bereitgestellt ist.

## Revendications

1. Dispositif pour soins de la peau comprenant :
- un bloc de tête (2) qui présente un vibreur ultrasonore (1) qui génère des vibrations ultrasonores ;
- un bloc de préhension (4) qui supporte le bloc de tête (2), le bloc de tête (2) étant appuyé sur une surface de la peau, en transmettant de ce fait les vibrations ultrasonores à la surface de la peau ; et
- un élément de raccordement (31) qui sert de mécanisme de support (7) afin de supporter le bloc de tête (2) sur le bloc de préhension (4) ;
dans lequel le bloc de tête (2) est supporté sur le bloc de préhension (4) par l'intermédiaire de l'élément de raccordement (31) de telle manière que le bloc de tête (2) ait la possibilité de se déplacer en allant en se rapprochant et en allant en s'éloignant du bloc de préhension (4) ;
**caractérisé en ce que** :
- l'élément de raccordement (31) sert en outre à permettre au bloc de tête (2) de tourner dans les trois dimensions dans toutes les directions par rapport ou bloc de préhension (4) ; et
- un dispositif d'arrêt anti-glissement (36) est couplé à une partie inférieure du bloc de tête (2), le dispositif d'arrêt anti-glissement (36) étant inséré dans un logement (20) du bloc de préhension (4) et saisi par la face intérieure du logement (20), grâce à quoi l'élément de raccordement est un élément élastique (31) qui est maintenu dans un état comprimé de manière à permettre à l'élément élastique (31) d'exercer une force de sollicitation dans une direction telle que le bloc de tête (2) se déplace en allant s'éloignant du bloc de préhension (4).

2. Dispositif pour soins de la peau selon la revendication 1, dans lequel un couvercle imperméable à l'eau (40) est interposé entre le logement (20) du bloc de préhension (4) et le dispositif d'arrêt (36).

3. Dispositif pour soins de la peau selon la revendication 1 ou la revendication 2, comprenant en outre un élément imperméable à l'eau (37) destiné à couvrir une circonférence extérieure de l'élément élastique (31) qui sert de mécanisme de support (7).

4. Dispositif pour soins de la peau selon l'une quelconque des revendications 1 à 3, dans lequel le bloc de tête (2) présente une surface inférieure constituée d'une surface convexe sphérique (42) et le bloc de préhension (4) présente une surface supérieure constituée d'une surface concave sphérique (43) qui fait face à la surface convexe (42) du bloc de tête (2) et dans lequel un intervalle (S) qui permet un déplacement du bloc de tête (2) est prévu entre la surface convexe (42) du bloc de tête (2) et la surface concave (43) du bloc de préhension (4).
